# EUROPÄISCHE PATENTSCHRIFT

(11) **EP 3 429 376 B1**
(45) Veröffentlichungstag und Bekanntmachung des Hinweises auf die Patenterteilung: **20.05.2026**
(21) Anmeldenummer: 16709809.4
(22) Anmeldetag: 14.03.2016
(51) Int. Cl.: A23L 27/16, A23L 23/10, A23L 27/10, A61K 36/8962, A23L 33/105, A23L 33/21, A61K 31/7004, A61K 31/7016, A61K 31/702, A61K 31/733, A23L 19/00, A23L 27/00

(54) **ZWIEBELSAFTKONZENTRAT**
ONION JUICE CONCENTRATE
CONCENTRÉ DE JUS D'OIGNONS

(43) Veröffentlichungstag der Anmeldung: 23.01.2019
(73) Patentinhaber: Symrise AG, 37603 Holzminden (DE)
(72) Erfinder: STÜRTZ, Melanie, 37671 Höxter (DE); KOCH, Jens, 37632 Eschershausen (DE); TILLMANN, Claudia, 37688 Beverungen (DE); SABATER-LUENTZEL, Christopher, 37603 Holzminden (DE)
(74) Vertreter: Eisenführ Speiser
(86) Internationale Anmeldenummer: PCT/EP2016/055479
(87) Internationale Veröffentlichungsnummer: WO 2017/157417

(56) Entgegenhaltungen:
- EP-A1- 1 227 738
- CN-A- 103 704 653
- BEN�TEZ VANESA ET AL: "The Impact of Pasteurisation and Sterilisation on Bioactive Compounds of Onion By-products", FOOD AND BIOPROCESS TECHNOLOGY ; AN INTERNATIONAL JOURNAL, vol. 6, no. 8, 8 May 2012 (2012-05-08), New York, pages 1979 - 1989, XP055775914, ISSN: 1935-5130, Retrieved from the Internet <URL:http://link.springer.com/article/10.1007/s11947-012-0866-x/fulltext.html> DOI: 10.1007/s11947-012-0866-x
- G BUFLER: "Qualit�t und Ertrag von �ko-Zwiebeln: Vergleich von Pflanzung und Direktsaat", TAGUNGSBAND DER 12. WISSENSCHAFTSTAGUNG �KOLOGISCHER LANDBAU: IDEAL UND WIRKLICHKEIT - PERSPEKTIVEN �KOLOGISCHER LANDBEWIRTSCHAFTUNG, 1 January 2013 (2013-01-01), Berlin, pages 348 - 351, XP093162516, ISBN: 978-3-89574-815-8, Retrieved from the Internet <URL:https://orgprints.org/id/eprint/21314/>
- ROLDÁN EDUVIGIS ET AL: "Characterisation of onion (Allium cepa L.) by-products as food ingredients with antioxidant and antibrowning properties", FOOD CHEMISTRY, vol. 108, no. 3, June 2008 (2008-06-01), pages 907 - 916, XP029166272, ISSN: 0308-8146, DOI: 10.1016/J.FOODCHEM.2007.11.058
- EDUVIGIS ROLDÁN-MARÍN ET AL: "Effects of an onion by-product on bioactivity and safety markers in healthy rats", BRITISH JOURNAL OF NUTRITION, vol. 102, no. 11, 17 August 2009 (2009-08-17), UK, pages 1574 - 1582, XP055290748, ISSN: 0007-1145, DOI: 10.1017/S0007114509990870
- NORIO SHIOMI ET AL: "Fructo-oligosaccharide content and fructosyltransferase activity during growth of onion bulbs", NEW PHYTOLOGIST, vol. 136, no. 1, 1 May 1997 (1997-05-01), GB, pages 105 - 113, XP055290433, ISSN: 0028-646X, DOI: 10.1111/j.1469-8137.1997.tb04736.x
- "Handbook of Vegetables and Vegetable Processing", 19 November 2010, JOHN WILEY & SONS, ISBN: 978-0-8138-1541-1, article WIESLAW WICZKOWSKI: "Garlic and Onion: Production, Biochemistry, and Processing", pages: 625 - 642, XP055290686

## Beschreibung

Die vorliegende Erfindung betrifft ein Zwiebelsaftkonzentrat, welches ein besonders vorteilhaftes Kohlenhydrat- und Geschmacks- bzw. Geruchstoffprofil aufweist, sowie ein Verfahren zur Herstellung eines solchen Zwiebelsaftkonzentrats.

Insbesondere betrifft die vorliegende Erfindung ein Zwiebelsaftkonzentrat mit einem besonders hohen Gehalt an Fructan und offenbart erstmals die damit verbundenen Vorteile in der Verarbeitbarkeit und der Verträglichkeit eines solchen Zwiebelsaftkonzentrats, die dessen Verwendung in der Nahrungsmittelindustrie besonders attraktiv machen.

Aufgrund ihres intensiven und vielseitigen Geschmacks sind Zwiebeln bzw. Zwiebelaromen eine der beliebtesten Zutaten bei der Zubereitung von Speisen. Je nach Zusammensetzung und Zubereitung können sie ein mild-würziges bis scharfes und/oder süßliches Aroma vermitteln. Charakteristisch für das Geschmacks- bzw. Geruchsprofil von Zwiebeln sind insbesondere die Abbauprodukte der schwefelhaltigen Aminosäure Isoalliin, die bei der Verarbeitung von Zwiebeln entstehen und in bestimmter Konzentration sogar eine die Schleimhäute reizende Wirkung - beispielsweise das Tränen der Augen beim Zerkleinern von Zwiebeln - hervorrufen können. Darüberhinaus ist eine große Vielfalt von Aromastoffen in Zwiebeln enthalten, die je nach Zusammensetzung das Aromaprofil beeinflussen und beispielweise einen zwiebligen, schwefligen, süßlichen und/oder adstringierenden Geschmackseindruck vermitteln oder verstärken können.

Zwiebeln werden üblicherweise entweder als einjährige Pflanzen angebaut, indem sie ausgesät und noch im gleichen oder im nächsten Jahr geerntet werden (Saatzwiebeln), oder im zweijährigen Verfahren, indem die im Vorjahr gezogenen Pflanzen erneut in den Boden gesetzt (gesteckt) werden (Steckzwiebeln).

Im Gegensatz zu vielen anderen Pflanzen speichern Zwiebeln keine Stärke, sondern Fructane als Reservekohlenhydrate. Fructane sind Oligo- oder Polysaccharide, die aus unterschiedlich verknüpften Fructofuranoseeinheiten, die an ein Saccharosemolekül gebunden sind, bestehen. Fructane aus der Zwiebel sind meist 1 bis 5-kettig und verzweigt mit einem Polymerisierungsgrand von 1 bis 9 und dabei gut wasserlöslich. Beispiele für typische Fructane aus der Zwiebel sind Nystose, 1-Kestose, 6-Kestose und Fructosylnystose.

Inulin ist ein Gemisch von Oligo- bzw. Polysacchariden aus Fructoseeinheiten, welches zu den Fructanen zählt und eine ganze Reihe von vorteilhaften Eigenschaften aufweist, die einen vielfältigen Einsatz in Nahrungsmitteln ermöglichen. Zunächst stellt es einen wertvollen Ballaststoff dar und kann andere Kohlenhydrate wie Stärke und - aufgrund seines süßlichen Geschmacks - auch Zucker in Nahrungsmitteln in kaloriensparender und die Verdauung fördernder Weise ersetzen. Darüberhinaus wird es aufgrund seiner cremig-sahnigen Konsistenz als Fettersatz in Magermilchprodukten verwendet und wirkt sich Studien zufolge günstig auf die Resorption von Mineralstoffen wie Calcium und Magnesium aus. Schließlich wurde für Inulin sogar eine präbiotische Wirkung beobachtet, wonach das Wachstum von nützlichen Darmbakterien gefördert wird. Bezüglich des Inulingehalts von bzw. in Zwiebeln siehe beispielsweise: Ernst et al., Characterization of Fructan Oligomers from Species of the Genus Allium L., J. Plant Physiol. Vol. 153, pp. 53-60 (1998); Niness, Inulin and Oligofructose: What Are They?, J Nutr. 1999 Jul;129(7 Suppl):1402S-6S.

CN103704653A betrifft einen konzentrierten Zwiebelsaft und dessen Herstellungsverfahren sowie ein nahrhaftes Mehrzweckgewürz, das zur Verbesserung des Geruchs von Fleisch und Fisch verwendet oder Suppen, Snacks und Gemüsesalaten zugesetzt werden kann.

Im Rahmen der vorliegenden Erfindung hat sich herausgestellt, dass ein Zwiebelsaftkonzentrat mit einem besonders hohen Gehalt an Fructan und einem insgesamt vorteilhaften Kohlenhydratprofil erhalten werden kann, wenn Steckwiebeln, mittels eines erfindungsgemäßen Verfahrens zu einem Zwiebelsaftkonzentrat verarbeitet werden.

Vorzugsweise lässt sich das damit erhaltene Zwiebelsaftkonzentrat auch besonders gut weiterverarbeiten, da es nicht anbrennt, nicht zu sehr schäumt und nicht klebt und da unerwünschte Bräunungen vermieden werden können.

Aufgabe der vorliegenden Erfindung war es, ein Zwiebelsaftkonzentrat bereitzustellen, welches eine besonders vorteilhafte Zusammensetzung aufweist.

Insbesondere war es Aufgabe der vorliegenden Erfindung, ein Zwiebelsaftkonzentrat mit einem Kohlenhydratprofil bereitzustellen, welches die oben beschriebenen vorteilhaften diätetischen Effekte verwirklicht.

Zudem war es Aufgabe der vorliegenden Erfindung, ein Zwiebelsaftkonzentrat mit einem Geschmacks- bzw. Geruchstoffprofil bereitzustellen, welches zum Einsatz als Geschmacks- und/oder Geruchsstoffmischung in Lebensmitteln geeignet ist.

Schließlich war es ebenfalls Aufgabe der vorliegenden Erfindung, ein Zwiebelsaftkonzentrat bereitzustellen, welches sich durch eine besonders gute Verarbeitbarkeit auszeichnet.

Diese Aufgaben werden erfindungsgemäß gelöst durch ein Zwiebelsaftkonzentrat (K), hergestellt durch das Verfahren der vorliegenden Erfindung und enthaltend 20 bis 45 Gew.-% Fructane, sowie 1 bis 22 Gew.-% Fructose, 2 bis 20 Gew.-% Glucose, 3 bis 25 Gew.-% Saccharose und 0,5 bis 10 Gew.-% Cellobiose, jeweils bezogen auf das Gesamtgewicht des Zwiebelsaftkonzentrats.

Das erfindungsgemäße Zwiebelsaftkonzentrat weist einen gegenüber herkömmlichen Zwiebelsaftkonzentraten erhöhten Gehalt an Fructan im Vergleich zu den Zuckern Fructose, Glucose und Saccharose auf.

Durch den erhöhten Fructangehalt wird die Verträglichkeit des erfindungsgemäßen Zwiebelsaftkonzentrats gesteigert. Wie einleitend bereits erwähnt, wirkt sich der hohe Gehalt an Oligo- bzw. Polyfruktosemolekülen positiv auf die Verdauung aus und kann zusätzliche gesundheitsfördernde Effekte wie eine verbesserte Mineralstoffresorption und eine präbiotische Wirkung mit sich bringen.

Die Korrelation von Zucker- bzw. Fructan-Gehalt zur Viskosität des Zwiebelsaftkonzentrats ergibt, dass ein höherer Zuckergehalt die Viskosität erniedrigt, während ein höherer Anteil an Fructan die Viskosität deutlich steigert. Wichtig ist insbesondere im Hinblick auf die Verarbeitbarkeit des Zwiebelsaftkonzentrats der Gehalt von Fructan und Fructose, da signifikant bessere Trockeneigenschaften erreicht werden, je größer das Verhältnis von Fructan zu Fructose ist.

Gemäß einer bevorzugten Ausgestaltung eines erfindungsgemäßen Zwiebelsaftkonzentrats (K) ist darin das Verhältnis des Gesamtgewichts von Fructan(en) zum Gesamtgewicht von Fructose 1 oder mehr, besonders bevorzugt 1,5 oder mehr.

Damit lässt sich das erfindungsgemäße Zwiebelsaftkonzentrat besonders gut weiterverarbeiten und in zahlreichen Nahrungsmittelprodukten bzw. Halbfertigwaren verschiedener Konsistenz verarbeiten. Vorzugsweise wird das Produkt gut und vollständig trocken ohne zu schäumen oder zu kleben. Vorteilhaft ist zudem, dass das erfindungsgemäße Zwiebelsaftkonzentrat vorzugsweise wesentlich heller, d.h. weniger orange-braun ist, da es beim Verarbeiten bzw. Trocknen vorzugsweise nicht bräunt und daher die Nahrungsmittelprodukte in denen es eingesetzt wird nicht unerwünscht verfärbt.

Das erfindungsgemäße Zwiebelsaftkonzentrat (K) ist vorzugsweise dadurch gekennzeichnet, dass es einen, zwei, mehrere oder sämtliche der Aromastoffe aus der folgenden Gruppe enthält: 2-Methyl-2-pentenal, Propanal, Hexanal, 2E-Heptenal, Dipropyldisulfid, Dipropyltrisulfid, Methylpropyldisulfid, Dimethyldisulfid, Dimethyltrisulfid, 1Z-Propenyl-propyldisulfid, 1E-Propenylpropyldisufid, cis-Methyl-1-propenyldisulfid, trans-Methyl-1-propenyldisulfid, Propanthiol und 2-Methyl-butyraldehyd.

Zwiebeln enthalten eine ganze Reihe von Aromastoffen, die ein vielseitiges Geschmacks- und Geruchsstoffprofil vermitteln können. Besonders charakteristisch sind die oben genannten Aromastoffe, die zum Teil erst bei der Verarbeitung von Zwiebeln entstehen und das Gesamtaroma variabel beeinflussen können.

Vorzugsweise enthält ein erfindungsgemäßes Zwiebelsaftkonzentrat (K) zudem einen oder mehrere Substanzen ausgewählt aus der Gruppe bestehend aus Zimtsäureamide und S-alkylierte Cysteinderivate, beispielsweise S-Alk(en)yl-cysteinsulfoxide und S-Alk(en)yl-cysteine.

Besonders vorteilhaft ist die Verwendung eines erfindungsgemäßen Zwiebelsaftkonzentrats (K) als Geschmacks- und/oder Geruchsstoffmischung oder als Bestandteil davon.

Dabei ist insbesondere die Verwendung zum Vermitteln oder Verstärken eines, mehrerer oder sämtlicher der folgenden Geschmacks- und/oder Geruchseindrücke: adstringierend, schwefelig, zwieblig, zwieblig frisch, süß, ölig, bitter, leicht röstig, leicht karamelig, scharf, beißend, erdig, grün (Richtung Apfel), grün (Richtung Gras), lauchig, fettig, frisch, frittiert, umami, vollmundig und gebraten bevorzugt.

Die vorliegende Offenlegung betrifft demgemäß auch eine Geschmacks- und/oder Geruchsstoffmischung, enthaltend ein Zwiebelsaftkonzentrat (K) wie vorstehend beschrieben.

Eine solche Geschmacks- und/oder Geruchsstoffmischung eignet sich für eine breite Anwendung in Würz- und Aromatisierungsprodukten, insbesondere solchen mit Brat- und Röstaromen, die in verschiedener Form, z.B. flüssig, als Pulver oder gepresst bzw. portioniert zum Auflösen, eingesetzt werden können. Insbesondere bevorzugt ist die Anwendung in trockener Form zum Beispiel als Pulver.

Ferner können die offengelegten Geschmacks- und/oder Geruchsstoffmischungen übliche Hilfsstoffe, weitere Würz- und Aromastoffe sowie Geschmacksverstärker enthalten.

Die vorliegende Offenlegung betrifft auch eine der Ernährung oder dem Genuss dienende Zubereitung, enthaltend ein erfindungsgemäßes Zwiebelsaftkonzentrat (K) oder eine offengelegte Geschmacks- und/oder Geruchsstoffmischung, vorzugsweise wobei die Gesamtmenge an in der Zubereitung enthaltenem Zwiebelsaftkonzentrat (K) ausreicht, um einen oder mehrere Geschmacks- und/oder Geruchseindrücke zu vermitteln oder zu verstärken, bevorzugt einen, mehrere oder sämtliche der folgenden Geschmacks- bzw. Geruchseindrücke: adstringierend, schweflig, zwieblig, zwieblig frisch, süß, ölig, bitter, leicht röstig, leicht karamelig, scharf, beißend, erdig, grün (Richtung Apfel), grün (Richtung Gras), lauchig, fettig, frisch, frittiert, umami, vollmundig, gebraten. Zudem kann das erfindungsgemäße Zwiebelsaftkonzentrat den Eindruck einer Erhöhung der Mundfülle bei würzigen Aromen hervorrufen.

Solche Zubereitungen können sowohl verzehrfertige Produkte als auch Halbfertigwaren sein.

Wie eingangs bereits erwähnt betrifft die vorliegende Erfindung auch Verfahren zur Herstellung eines Zwiebelsaftkonzentrats (K) aus Steckzwiebeln, umfassend die oder bestehend aus den folgenden Schritte(n):
(a) Bereitstellen einer Zwiebelmaische (M),
(b) Zubereiten eines Zwiebelsafts (S) aus der Zwiebelmaische (M),
(c) Trennung der Zwiebelmaische (M) von Zwiebelsaft (S) und Trester,
(d) optional Einstellen des pH-Werts des Zwiebelsafts (S) auf einen pH im Bereich von 3 bis 7, vorzugsweise von 4 bis 6, besonders bevorzugt von 4,5 bis 5,5,
(e) Klärung des Zwiebelsaftes,
(f) Zubereiten eines Zwiebelsaftkonzentrats (K) aus dem Zwiebelsaft (S) um ein Zwiebelsaftkonzentrat (K) enthaltend 20 bis 45 Gew.-% Fructane, sowie 1 bis 22 Gew.-% Fructose, 2 bis 20 Gew.-% Glucose, 3 bis 25 Gew.-% Saccharose und 0,5 bis 10 Gew.-% Cellobiose, jeweils bezogen auf das Gesamtgewicht des Zwiebelsaftkonzentrats zu erhalten,
wobei Schritt (b) die folgenden Teilschritte umfasst oder daraus besteht:
(b1) Erhitzen der Zwiebelmaische (M) auf 30 bis 60 °C, vorzugsweise auf 40 bis 55 °C, besonders bevorzugt auf 48 bis 52 °C,
(b2) Zusetzen einer Enzymmischung zur Zwiebelmaische (M),
(b3) Inkubieren der Zwiebelmaische (M) für 1 Stunde oder mehr, vorzugsweise für 2 Stunden oder mehr.

Im Kontext der vorliegenden Offenlegung deckt der Begriff "Zwiebel" sämtliche bekannten Sorten ab, insbesondere gehören dazu Speisezwiebeln, Gemüsezwiebeln, rote und weiße Zwiebeln, Schalotten, Lauchzwiebeln und Silberzwiebeln. Diese können im einjährigen oder zweijährigen Verfahren gezogen worden sein (Saatzwiebeln oder Steckzwiebeln).

Unter Zwiebelmaische (M) ist im Zusammenhang mit der vorliegenden Erfindung das (vor-)zerkleinerte und gegebenenfalls (teilweise) gereinigte Zwiebelmaterial zu verstehen, welches in dem erfindungsgemäßen Verfahren verarbeitet wird.

Der Zwiebelsaft (S) umfasst sämtliche aus dem Zwiebelmaterial freigesetzte, im Wesentlichen flüssige Substanzen, die in Schritt (c) von den vorwiegend festen Rückständen (Trester) getrennt werden.

"Trennen" umfasst im Kontext der vorliegenden Erfindung alle dem Fachmann geläufigen Verfahren zum (teilweisen) Separieren verschiedener Bestandteile einer Mischung. Insbesondere sind hierzu Verfahren wie Pressen oder Filtrieren zum Trennen der Zwiebelmaische von Zwiebelsaft und Trester geeignet.

Die "Klärung" gemäß Schritt (e) des erfindungsgemäßen Verfahrens kann mittels jedem dem Fachmann geläufigen Verfahren bzw. jeder Vorrichtung zum (teilweisen) Entfernen von Verunreinigungen erfolgen. Beispielsweise können hierfür Fällung, Filtration, Ultrafiltration, Mikrofiltration, reverse Osmose oder die Verwendung von Separatoren oder Dekantern zur Klärung des Zwiebelsaftes beitragen.

Das durch das erfindungsgemäße Verfahren hergestellte Zwiebelsaftkonzentrat weist eine wie oben beschriebene vorteilhafte Zusammensetzung sowohl hinsichtlich des Zucker- bzw. Fructangehaltes als auch hinsichtlich des Geschmacks- bzw. Geruchsstoffprofils auf.

Zudem ist es vorteilhafterweise besonders gut weiterverarbeitbar, da es vorzugsweise nicht klebt, anbrennt oder zu sehr schäumt und eine hellere Färbung (weniger orange-braun) als herkömmliche Zwiebelsaftkonzentrate aufweist. Bevorzugt ist erfindungsgemäß ein Verfahren, welches dadurch gekennzeichnet ist, dass Schritt (a) die folgenden Teilschritte umfasst oder daraus besteht:
(a1) Bereitstellen und optional waschen von Steckzwiebeln,
(a2) Zerkleinern der Zwiebeln aus Schritt (a1).

Die Bereitstellung der Maische kann das Waschen der Zwiebeln, sowie das Entfernen von Erde, Steinen, sowie optional Wurzeln, Trieben oder Schalen, sowie mehrere Zerkleinerungsschritte, z.B. Vorzerkleinerung und anschließendes Vermahlen, umfassen. Darüberhinaus können faulige oder kranke Zwiebeln gezielt aussortiert werden.

Das erfindungsgemäße Verfahren ist dadurch gekennzeichnet, dass Schritt (b) die folgenden Teilschritte umfasst oder daraus besteht:
(b1) Erhitzen der Zwiebelmaische (M) auf 30 bis 60 °C, vorzugsweise auf 40 bis 55 °C, besonders bevorzugt auf 48 bis 52 °C,
(b2) Zusetzen einer Enzymmischung zur Zwiebelmaische (M),
(b3) Inkubieren der Zwiebelmaische (M) für 1 Stunde oder mehr, vorzugsweise für 2 Stunden oder mehr.

Der Einsatz bestimmter Enzyme kann den Aufschluss des Zwiebelmaterials beispielsweise durch gezielten Abbau von Zellwänden verbessern. Die dabei gebildeten Abbauprodukte wirken sich vorteilhaft - wie oben beschrieben - auf die Kohlenhydrat-Zusammensetzung des entstehenden Zwiebelsaftes aus. Zudem wird durch den effizienten Abbau die Freisetzung bzw. Entstehung (durch Reaktion) von Aromastoffen gefördert, was zu einem besonders intensiven Aroma des entstehenden Zwiebelsaftes führt.

Geeignete Enzyme sind in diesem Zusammenhang beispielsweise Carbohydrasen. Dieser Oberbegriff umfasst Enzyme, die den Abbau von einfachen bis zu sehr komplexen Kohlenhydraten, wie sie z.B. in Zellwänden von Pflanzen vorkommen, katalysieren können. Dabei entstehen verschiedene Poly-und Oligosaccharide und diese werden gegebenenfalls bis zu Di- und Monosacchariden (einfachen Zuckern) weiter abgebaut.

Beispiele für geeignete Carbohydrasen die in dem erfindungsgemäßen Verfahren eingesetzt werden können sind Arabanase, Cellulase, beta-Glucanase, Hemicellulase, Xylanase und Pectinase. Die Verwendung von einer Mischung umfassend, zwei, mehrere oder sämtliche diese Enzyme führt - wie sich gezeigt hat - insbesondere zu der erfindungsgemäß vorteilhaften Zusammensetzung des hergestellten Zwiebelsaftkonzentrats.

Gemäß einer bevorzugten Ausgestaltung des erfindungsgemäßen Verfahrens enthält die Enzymmischung daher eine oder mehrere Carbohydrasen, wobei die bzw. eine Carbohydrase oder mehrere oder sämtliche der Carbohydrasen vorzugsweise ausgewählt ist/sind aus der Gruppe bestehend aus: Arabanase, Cellulase, beta-Glucanase, Hemicellulase, Xylanase und Pectinase.

Weiterhin bevorzugt ist ein erfindungsgemäßes Verfahren, welches dadurch gekennzeichnet ist, dass Schritt (f) die folgenden Teilschritte umfasst oder daraus besteht:
(f1) Pasteurisieren durch Injektion von Wasserdampf in den Zwiebelsaft (S),
(f2) optional Hinzufügen weiterer Bestandteile zum Zwiebelsaft (S),
(f3) Entfernen mindestens eines Teils des Wassers aus dem Zwiebelsaft (S), um ein Zwiebelsaftkonzentrat (K) von mindestens 30 ° Brix, bevorzugt 65 bis 85 ° Brix zu erhalten.

Um ein nahrungsmitteltaugliches bzw. haltbares Zwiebelsaftkonzentrat zu erhalten, wird der Zwiebelsaft zunächst pasteurisiert. Hierzu sind dem Fachmann verschiedene Techniken zum kurzzeitigen Erhitzen auf Temperaturen, bei denen Mikroorganismen (teilweise) abgetötet werden, geläufig. Besonders bevorzugt ist bei dem erfindungsgemäßen Verfahren die Injektion von Wasserdampf, da diese an dieser Stelle des Verfahrens leicht und effizient technisch umsetzbar und zudem besonders energieeffizient ist.

Optional können dem erhaltenen Zwiebelsaft weitere Bestandteile zugesetzt werden, damit diese mit dem Zwiebelsaft intensiv vermischt werden können bevor er der Konzentrierung unterzogen wird.

Das Entfernen mindestens eines Teils des Wassers aus dem Zwiebelsaft um ein Zwiebelsaftkonzentrat (K) von mindestens 30 ° Brix, bevorzugt 65 bis 85 ° Brix zu erhalten, erfolgt bevorzugt mittels Vakuumbandtrocknung, Sprühtrocknung, Gefriertrocknung, Roller drying, im Wirbelschicht- oder Vakuumdünnschichtverdampfer, mittels anderer Vakuumverfahren oder Membrantechnologien sowie durch Bratung bzw. Verwendung von Plattenkondensatoren. Durch bestimmte Trocknungsverfahren wie zum Beispiel dem Braten kann es vorteilhafterweise zur Entstehung weiterer Reaktionsaromen und damit zur Intensivierung des Gesamtaromas des erfindungsgemäß hergestellten Zwiebelsaftkonzentrats kommen.

Die Angaben in ° Brix beziehen sich auf die Dichte des Zwiebelsaftkonzentrats, wobei die Dichte derjenigen Dichte einer Lösung von Saccharose in Wasser entspricht, die so viele Gramm Saccharose pro 100 g Lösung enthält, wie die Angabe in ° Brix nennt. Das Zwiebelsaftkonzentrat hat beispielsweise 1 ° Brix, wenn es dieselbe Dichte wie eine Lösung von 1 g Saccharose in 100 g Saccharose/Wasser-Lösung hat. 10 ° Brix entsprechen demnach einer Dichte von 10 g Saccharose in 100 g Saccharose/Wasser-Lösung.

Schließlich betrifft die vorliegende Erfindung auch ein Zwiebelsaftkonzentrat wie oben definiert, hergestellt oder herstellbar durch ein Verfahren wie in den vorhergehenden Abschnitten beschrieben.

Für ein nach dem erfindungsgemäßen Verfahren hergestelltes oder herstellbares Zwiebelsaftkonzentrat gilt das bereits oben im Zusammenhang mit dem erfindungsgemäßen Zwiebelsaftkonzentrat Gesagte entsprechend. Insbesondere weist es die vorteilhafte Zusammensetzung sowie die besonders gute Verarbeitbarkeit - wie oben beschrieben - auf.

Die folgenden Beispiele sollen dazu dienen, die vorliegende Erfindung näher zu charakterisieren ohne dabei jedoch den Gegenstand einzuschränken. Soweit nicht anders angegeben, beziehen sich dabei alle Prozentangaben auf das Gewicht.

### Beispiele:

### Beispiel 1: Herstellung von Zwiebelsaftkonzentrat:

Gemäß hierin bzw. oben beschriebenem Verfahren: Zwiebeln werden gewaschen, zerkleinert zu einer Maische, woraus der Zwiebelsaft abgetrennt wird. Nach Einstellen auf einen pH von ca. 5 wird dieser Saft geklärt und entsprechend dem hierin bzw. oben beschriebenen Verfahren weiter zu Zwiebelsaftkonzentrat verarbeitet, das eine erfindungsgemäße Zusammensetzung aufweist. Dieses Zwiebelsaftkonzentrat kann sowohl pur als auch in Mischungen mit Trägermaterialien oder verschiedenen Ölen verwendet werden.

### Beispiel 2: Anwendungsbeispiele (nicht gemäß der vorliegenden Erfindung):

### 2.1 Frische Zwiebelnote (sprühgetrocknet):

29 % Zwiebelsaftkonzentrat werden mit Wasser, Zwiebelöl und einem Trägerstoff dispergiert. Die erhaltene Slurry wird unter ständigem Rühren sprühgetrocknet.

### 2.2 Sautiertes Zwiebelprofil (Paste):

58 % Zwiebelsaftkonzentrat und Apfelsaft werden zusammen mit Butter gemischt, erhitzt und homogenisiert.

### 2.3 Bratzwiebelprofil (Paste):

80 % Zwiebelsaftkonzentrat werden mit Pflanzenöl gemischt, erhitzt und homogenisiert.

### 2.4 Gekochtes Zwiebelprofil (viskose Flüssigkeit):

90% Zwiebelsaftkonzentrat werden mit Proteinbausteinen und Fruchtzucker gemischt, erhitzt und homogenisiert.

## Patentansprüche

1. Verfahren zur Herstellung eines Zwiebelsaftkonzentrats (K) aus Steckzwiebeln, umfassend die oder bestehend aus den folgenden Schritte(n):
(a) Bereitstellen einer Zwiebelmaische (M),
(b) Zubereiten eines Zwiebelsafts (S) aus der Zwiebelmaische (M),
(c) Trennung der Zwiebelmaische (M) von Zwiebelsaft (S) und Trester,
(d) optional Einstellen des pH-Werts des Zwiebelsafts (S) auf einen pH im Bereich von 3 bis 7, vorzugsweise von 4 bis 6, besonders bevorzugt von 4,5 bis 5,5,
(e) Klärung des Zwiebelsaftes,
(f) Zubereiten eines Zwiebelsaftkonzentrats (K) aus dem Zwiebelsaft (S) um ein Zwiebelsaftkonzentrat (K) enthaltend 20 bis 45 Gew.-% Fructane, sowie 1 bis 22 Gew.-% Fructose, 2 bis 20 Gew.- % Glucose, 3 bis 25 Gew.-% Saccharose und 0,5 bis 10 Gew.-% Cellobiose, jeweils bezogen auf das Gesamtgewicht des Zwiebelsaftkonzentrats zu erhalten,
wobei Schritt (b) die folgenden Teilschritte umfasst oder daraus besteht:
(b1) Erhitzen der Zwiebelmaische (M) auf 30 bis 60 °C, vorzugsweise auf 40 bis 55 °C, besonders bevorzugt auf 48 bis 52 °C,
(b2) Zusetzen einer Enzymmischung zur Zwiebelmaische (M),
(b3) Inkubieren der Zwiebelmaische (M) für 1 Stunde oder mehr, vorzugsweise für 2 Stunden oder mehr.

2. Verfahren nach Anspruch 1, **dadurch gekennzeichnet, dass** Schritt (a) die folgenden Teilschritte umfasst oder daraus besteht:
(a1) Bereitstellen und optional Waschen von Steckzwiebeln,
(a2) Zerkleinern der Zwiebeln aus Schritt (a1).

3. Verfahren nach Anspruch 1 oder 2, **dadurch gekennzeichnet, dass** Schritt (f) die folgenden Teilschritte umfasst oder daraus besteht:
(f1) Pasteurisieren durch Injektion von Wasserdampf in den Zwiebelsaft (S),
(f2) optional Hinzufügen weiterer Bestandteile zum Zwiebelsaft (S),
(f3) Entfernen mindestens eines Teils des Wassers aus dem Zwiebelsaft (S), um ein Zwiebelsaftkonzentrat (K) von mindestens 30 ° Brix, bevorzugt 65 bis 85 ° Brix zu erhalten.

4. Verfahren nach einem der Ansprüche 1 bis 3, **dadurch gekennzeichnet, dass** die Enzymmischung eine oder mehrere Carbohydrasen enthält.

5. Verfahren nach Anspruch 4, **dadurch gekennzeichnet, dass** die bzw. eine Carbohydrase oder mehrere oder sämtliche der Carbohydrasen ausgewählt ist/sind aus der Gruppe bestehend aus: Arabanase, Cellulase, beta-Glucanase, Hemicellulase, Xylanase und Pectinase.

6. Zwiebelsaftkonzentrat (K) enthaltend 20 bis 45 Gew.-% Fructane, sowie 1 bis 22 Gew.-% Fructose, 2 bis 20 Gew.-% Glucose, 3 bis 25 Gew.-% Saccharose und 0,5 bis 10 Gew.-% Cellobiose, jeweils bezogen auf das Gesamtgewicht des Zwiebelsaftkonzentrats, hergestellt oder herstellbar durch ein Verfahren nach einem der Ansprüche 1 bis 5.

7. Zwiebelsaftkonzentrat nach Anspruch 6, **dadurch gekennzeichnet, dass** das Zwiebelsaftkonzentrat (K) einen, zwei, mehrere oder sämtliche der Aromastoffe aus der folgenden Gruppe enthält: 2-Methyl-2-pentenal, Propanal, Hexanal, 2E-Heptenal, Dipropyldisulfid, Dipropyltrisulfid, Methylpropyldisulfid, Dimethyldisulfid, Dimethyltrisulfid, 1Z-Propenyl-propyldisulfid, 1E-Propenylpropyldisufid, cis-Methyl-1-propenyldisulfid, trans-Methyl-1-propenyldisulfid, Propanthiol und 2-Methylbutyraldehyd.

8. Zwiebelsaftkonzentrat (K) nach Anspruch 6 oder 7, zudem enthaltend einen oder mehrere weitere Substanzen ausgewählt aus der Gruppe bestehend aus Zimtsäureamide und S-alkylierte Cysteinderivate, beispielsweise S-Alk(en)yl-cys-teinsulfoxide und S-Alk(en)yl-cysteine.

## Claims

1. Method for producing an onion juice concentrate (K) from onion sets, comprising or consisting of the following step(s):
(a) providing an onion mash (M),
(b) preparing an onion juice (S) from the onion mash (M),
(c) separating the onion mash (M) from onion juice (S) and pomace,
(d) optionally adjusting the pH value of the onion juice (S) to a pH in the range of 3 to 7, preferably 4 to 6, more preferably 4.5 to 5.5,
(e) clarifying the onion juice,
(f) preparing an onion juice concentrate (K) from the onion juice (S) so as to obtain an onion juice concentrate (K) containing 20 to 45 wt.% fructans, 1 to 22 wt.% fructose, 2 to 20 wt.% glucose, 3 to 25 wt.% sucrose, and 0.5 to 10 wt.% cellobiose, in each case based on the total weight of the onion juice concentrate,
wherein step (b) comprises or consists of the following sub-steps:
(b1) heating the onion mash (M) to 30 to 60 °C, preferably to 40 to 55 °C, more preferably to 48 to 52 °C,
(b2) adding an enzyme mixture to the onion mash (M),
(b3) incubating the onion mash (M) for 1 hour or more, preferably for 2 hours or more.

2. The method according to claim 1, **characterized in that** step (a) comprises or consists of the following sub-steps:
(a1) providing and optionally washing onion sets,
(a2) comminuting the onions from step (a1).

3. The method according to claim 1 or 2, **characterized in that** step (f) comprises or consists of the following sub-steps:
(f1) pasteurizing by injection of steam into the onion juice (S),
(f2) optionally adding further constituents to the onion juice (S),
(f3) removing at least a portion of the water from the onion juice (S) to obtain an onion juice concentrate (K) having at least 30 °Brix, preferably 65 to 85 °Brix.

4. The method according to any one of claims 1 to 3, **characterized in that** the enzyme mixture comprises one or more carbohydrases.

5. The method according to claim **4, characterized in that** the carbohydrase or one of the carbohydrases or two or more or all of the carbohydrases is/are selected from the group consisting of: arabanase, cellulase, beta-glucanase, hemicellulase, xylanase, and pectinase.

6. Onion juice concentrate (K) containing 20 to 45 wt.% fructans, 1 to 22 wt.% fructose, 2 to 20 wt.% glucose, 3 to 25 wt.% sucrose, and 0.5 to 10 wt.% cellobiose, in each case based on the total weight of the onion juice concentrate, produced or producible by a method according to any one of claims 1 to 5.

7. The onion juice concentrate according to claim 6, **characterized in that** the onion juice concentrate (K) contains one, two, more, or all of the flavor compounds from the following group: 2-methyl-2-pentenal, propanal, hexanal, 2E-heptenal, dipropyl disulfide, dipropyl trisulfide, methyl propyl disulfide, dimethyl disulfide, dimethyl trisulfide, 1Z-propenyl propyl disulfide, 1E-propenyl propyl disulfide, cis-methyl-1-propenyl disulfide, trans-methyl-1-propenyl disulfide, propanethiol, and 2-methyl-butyraldehyde.

8. The onion juice concentrate (K) according to claim 6 or 7, further containing one or more additional substances selected from the group consisting of cinnamic acid amides and S-alkylated cysteine derivatives, for example S-alk(en)yl cysteine sulfoxides and S-alk(en)yl cysteines.

## Revendications

1. Procédé de production d'un concentré de jus d'oignon (K) à partir d'oignons de semence, comprenant les ou constitué des étapes suivantes consistant à:
(a) fournir une purée d'oignons (M),
(b) préparer un jus d'oignon (S) à partir de la purée d'oignons (M),
(c) séparer la purée d'oignons (M) du jus d'oignon (S) et du marc,
(d) en option, ajuster la valeur pH du jus d'oignon (S) à une valeur pH comprise entre 3 et 7, de préférence entre 4 et 6, de manière particulièrement préférée entre 4,5 et 5,5,
(e) clarifier le jus d'oignon,
(f) préparer un concentré de jus d'oignon (K) à partir du jus d'oignon (S) afin d'obtenir un concentré de jus d'oignon (K) contenant de 20 à 45 % en poids de fructanes, ainsi que de 1 à 22 % en poids de fructose, de 2 à 20 % en poids de glucose, de 3 à 25 % en poids de saccharose et de 0,5 à 10 % en poids de cellobiose, respectivement par rapport au poids total du concentré de jus d'oignon,
dans lequel l'étape (b) comprend les ou est constituée des sous-étapes suivantes consistant à:
(b1) chauffer la purée d'oignons (M) à une température de 30 à 60 °C, de préférence de 40 à 55 °C, de manière particulièrement préférée de 48 à 52 °C,
(b2) ajouter un mélange enzymatique à la purée d'oignons (M),
(b3) incuber la purée d'oignons (M) pour une heure ou plus, de préférence pour deux heures ou plus.

2. Procédé selon la revendication 1, **caractérisé par le fait que** l'étape (a) comprend les ou est constituée des sous-étapes suivantes consistant à :
(a1) fournir et, en option, laver les oignons de semence ;
(a2) hacher les oignons de l'étape (a1).

3. Procédé selon la revendication 1 ou 2, **caractérisé par le fait que** l'étape (f) comprend les ou est constituée des sous-étapes suivantes consistant à :
(f1) pasteuriser par injection de la vapeur d'eau dans le jus d'oignon (S),
(f2) en option, ajouter d'autres composants au jus d'oignon (S),
(f3) éliminer au moins une partie de l'eau du jus d'oignon (S) pour obtenir un concentré de jus d'oignon (K) d'au moins 30 ° Brix, de préférence compris entre 65 et 85 ° Brix.

4. Procédé selon l'une quelconque des revendications 1 à 3, **caractérisé par le fait que** le mélange enzymatique contient une ou plusieurs carbohydrase(s).

5. Procédé selon la revendication 4, **caractérisé par le fait que** la ou bien les carbohydrase(s) ou plusieurs ou toutes les carbohydrases est/sont choisie(s) dans le groupe constitué par: l'arabanase, la cellulase, la bêta-glucanase, l'hémicellulase, la xylanase et la pectinase.

6. Concentré de jus d'oignon (K) contenant de 20 à 45 % en poids de fructanes, ainsi que de 1 à 22 % en poids de fructose, de 2 à 20 % en poids de glucose, de 3 à 25 % en poids de saccharose et de 0,5 à 10 % en poids de cellobiose, respectivement par rapport au poids total du concentré de jus d'oignon, produit ou pouvant être produit par un procédé selon l'une quelconque des revendications 1 à 5.

7. Concentré de jus d'oignon selon la revendication 6, **caractérisé par le fait que** le concentré de jus d'oignon (K) contient un, deux, plusieurs ou toutes les substances aromatisantes du groupe suivant : 2-méthyl-2-penténal, propanal, hexanal, 2E-hepténal, disulfure de dipropyle, trisulfure de dipropyle, disulfure de méthylpropyle, disulfure de diméthyle, trisulfure de diméthyle, disulfure de 1Z-propénylpropyle, disulfure de 1E-propénylpropyle, disulfure de cis-méthyl-1-propényle, disulfure de trans-méthyl-1-propényle, propanethiol et 2-méthylbutyraldéhyde.

8. Concentré de jus d'oignon (K) selon la revendication 6 ou 7, comprenant en outre une ou plusieurs autres substances choisies dans le groupe constitué par les amides de l'acide cinnamique et les dérivés de cystéine S-alkylés, par exemple les sulfoxydes de S-alk(én)yl-cystéine et les S-alk(én)ylcystéines.
